# EUROPEAN PATENT SPECIFICATION

(11) **EP 0 504 321 B1**
(45) Date of publication and mention of the grant of the patent: **07.05.1997**
(21) Application number: 91902823.3
(22) Date of filing: 29.11.1990
(51) Int. Cl.: C12Q 1/68, C07H 21/00

(54) **ENHANCED CAPTURE OF TARGET NUCLEIC ACID BY THE USE OF OLIGONUCLEOTIDES COVALENTLY ATTACHED TO POLYMERS**
VERSTÄRKTER EINFANG VON ZIEL-NUKLEINSÄUREN MITTELS VERWENDUNG VON OLIGONUKLEOTIDEN, DIE KOVALENT AN POLYMERE GEBUNDEN SIND
CAPTURE AMELIOREE D'ACIDE NUCLEIQUE CIBLE FAISANT APPEL A DES OLIGONUCLEOTIDES ATTACHES DE FA ON COVALENTE A DES POLYMERES

(30) Priority: 04.12.1989 US 444872
(43) Date of publication of application: 23.09.1992
(73) Proprietor: MICROPROBE CORPORATION, Bothell, WA 98021 (US)
(72) Inventor: VAN NESS, Jeffrey, Bothell, WA 98011 (US)
(74) Representative: Thomson, Paul Anthony
(86) International application number: US9006947
(87) International publication number: WO9108307

(56) References cited:
- EP-A- 0 159 719
- EP-A- 0 225 807
- EP-A- 0 265 244

## Description

### BACKGROUND OF THE INVENTION

### 1. Field of the Invention.

This invention relates to methods for detecting polynucleotide sequences using nucleic acid hybridization procedures. In particular, the invention concerns the use of oligonucleotides covalently attached to a polymer to increase the rate and extent of capture of specific target nucleic acid contained in a biological sample. In a preferred method, the target nucleic acid in the sample is exposed to a polynucleotide-capturing polymer having multiple oligonucleotide sequences complementary to sequences on the target. After capture of target polynucleotides, the polymer is immobilized (sequestered) onto a solid support through a ligand/receptor combination.

### 2. Information Disclosure.

Nucleic acid hybridization is a known and documented method for identifying nucleic acids. Hybridization is based on base pairing of complementary nucleic acid strands. When single stranded nucleic acids are incubated in appropriate buffer solutions, complementary base sequences pair to form stable double stranded molecules. The presence or absence of such pairing may be detected by different methods well known in the art.

Most hybridization assays involve multiple steps such as the hybridization technique described by Dunn & Hassell in Cell, Vol. 12, pages 23-36 (1977). Their assay is of the sandwich-type.

Ranki et al. in U.S. Patent No. 4,486,539 and 4,563,419 describe sandwich-type assays which first require steps to denature nucleic acids and then the single strand nucleic acids are allowed to hybridize with a nucleic acid affixed to a solid carrier and with a second nucleic acid labeled with a radioisotope.

Stabinsky, U.S. Patent No. 4,751,177 (Amgen) describes the use of a mediator oligonucleotide that hybridizes to both sequences on the target and the solid support. U.S. Patent No. 4,751,177 describes a linker that has only a single complementary sequence for each of the target and the solid support on the linker. A similar linker is used in EP A 225,807. Polymeric reagents have been described for amplification of signal in a variety of diagnostic assays (U.S. Patent Nos. 4,687,732 and 4,152,411).

EP-A-0 159 719 discloses a method of detecting target polynucleotides comprising contacting the target with polymer particles having a plurality of probes attached thereon and used in a process of agglutination by forming a multi hybrid with a plurality of target sequences. The polymer particles may be suspended or be in solution.

### SUMMARY OF THE INVENTION

This invention provides for a method of capturing target polynucleotides comprising:
(a) contacting the target polynucleotides with complementary capture polynucleotides covalently bound to polymers having at least two copies of the capture polynucleotides covalently bound;
(b) permitting hybridization to occur between the complementary polynucleotides; and
(c) binding the polymer to a solid surface.

It is preferred that the polymers be water soluble. The preferred water-soluble polymers are selected from the group consisting of polyoxides, polyethers, poly(ethylene imine), acrylic group polymers, vinyl group polymers and the polyvinylpyrrolidines. The most preferred polymers are selected from the group consisting of poly(ethylene imine), polyallylamine and polyvinylamine. The water soluble polymers preferably have a molecular weight of between about 600 and about 100,00 daltons, and most preferably a molecular weight of between about 10,000 and about 70,000 daltons.

For solid phase or mixed phase nucleic acid hybridization assays, the polymers may have a ligand bound thereto where the ligand has a corresponding binding receptor attached to a solid support through a covalent bond and the method described above would further comprise the binding of the water soluble polymer to the solid support. All signaling processes are detected on or in solution around the solid support. It is preferred that the ligand is a polynucleotide probe and that the receptor is a polynucleotide complementary to the probe.

The method may optionally utilize a microbead whereby a ternary complex of a microbead water soluble polymer and target polynucleotide is formed. This ternary complex may then be sequestered or bound to a two dimensional solid support. Preferred microbeads include those selected from the group consisting of magnetic beads and polystyrene/latex beads.

This invention also discloses a method of capturing target polynucleotides comprising:
(a) contacting the target polynucleotides with complementary capture nucleic acids covalently bound to microbeads having at least two copies of the capture polynucleotides covalently bound thereto;
(b) permitting hybridization to occur between the complementary nucleic acids;
(c) sequestering the microbeads onto a solid surface; and
(d) detecting the hybridization.

Preferred microbeads are selected from the group consisting of magnetic beads, teflon beads, silica and polystyrene/latex beads. It is preferred that the sequestering occur as a result of duplex formation between complementary oligonucleotides. It is further preferred that the microbeads have a diameter of about 0.5 to about 120 microns.

There is also disclosed herein compositions of target polynucleotide scavenging polymers comprising a water soluble polymeric backbones and at least two capture polynucleotides covalently attached wherein the polymeric backbone has a molecular weight of about 600 to about 100,000 daltons. It is preferred that the molecular weight is between about 10,000 and 70,000 daltons. The backbone is preferably selected from the same polymers provided above and even more preferably selected from the group consisting of poly(ethylene imine), polyallyl and polyvinylamine. The polymeric backbones are preferably covalently bound to at least two solid support-complementary capture nucleic acids. The preferred capture polynucleotides are oligonucleotides of an average of about 15 to about 70 bases in length.

The probes and methods of this invention can be used to detect target nucleic acid from any biological source but will find particular application in the detection of bacterial and viral organisms. The invention can be used in the detection of one or multiple targets. A preferred mode is the multiple target dipstick where a solid surface is used to capture polymers binding more than one type of target. These dipsticks have multiple discrete regions to which the capture polymers may bind and are designed to detect multiple targets such as a panel of infective viruses or bacteria.

### Definitions.

The term "microbead" will refer to polymers including crystals (such as silica) that are water insoluble in that they can be separated from a aqueous solution by centrifugation (centripetal force). The term microbead will also include microspheres.

The term "polymer" in this document will refer to both water soluble polymers and microbeads.

The term "polynucleotide" will refer to both long and short probes, the short probes may be referred to as oligonucleotides or oligos.

The term "sequestering" refers to the binding of the capture polymer to a solid support by ligand/receptor binding, typically by nucleic acid duplex formation.

The term "solid support" refers to any surface which is transferable from solution to solution and includes membranes, beads, microtiter wells, strings, plastic strips or any surface onto which DNA probes may be immobilized.

### FIGURES

Figure 1 provides a cartoon of the target scavenging polymer in operation.

Figure 2 provides a description of the kinetics of hybridization with or without the employment of "capture" or scavenging polymer.

### DETAILED DESCRIPTION

This invention provides improved methods for conducting nucleic acid hybridization assays. The improvement involves the enhanced capture of target nucleic acid from a solution using polymers onto which multiple oligonucleotides are covalently attached. For solid phase assays, the use of these polymers dramatically decreases the time required to sequester target nucleic acid onto a solid support and increases the quantity of target nucleic acid sequestered on the solid support when compared to assays not using this method. A microbead may be substituted for a water soluble polymer.

In the typical nucleic acid hybridization assay using a sandwich format, three nucleotide sequences participate in the assay. A capture probe which is typically bound to a solid support functions as a means to capture the target nucleic acid. The target nucleic acid which is typically in free solution functions as the nucleic acid being detected as being present or absent. Thirdly, a signal probe is used. Signal probes are typically bound to a detectable entity, e.g., an enzyme or radioisotope, which functions as a means to signal the presence or absence of the target nucleic acid. The capture and signal probes are complementary to different sequences of the target nucleic acid. The sandwich assay arises from the interaction of three nucleic acids hybridizing with each other to form a three layered composition where the capture and signal probes act as layers forming a sandwich around the target probe.

In this invention, a fourth component is introduced to enhance the rate of hybridization without the requirement to introduce any additional steps in the assay. The fourth component is the polynucleotide bound polymer. Although the means for binding the polymer to the solid support may be any ligand/receptor combination, oligonucleotide duplex formation is preferred. The oligonucleotides that are covalently attached to the polymer can be identical to each other or different. Where the oligonucleotides bound to a polymer represents only one oligonucleotide base sequence, that sequence will bind to both the target and the solid support. The only stoichiometric considerations being that the quantity of capture polymer employed does not exceed the amount of polymer that can be possibly be captured by oligonucleotide on the solid support.

### A. Polymers

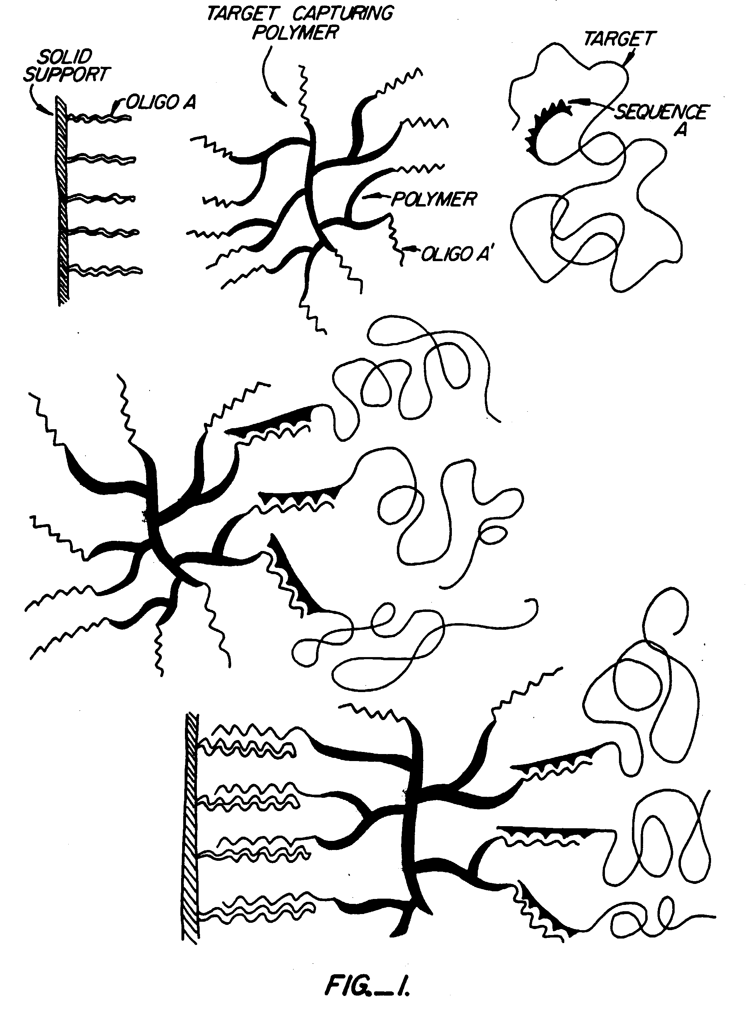

The polymers of this invention are free in solution and their relative mobility optimizes the kinetics for capturing target nucleic acid. Once the target is bound to the polymer, the polymer is permitted to bind (usually hybridize) to a solid support. This second step occurs with very rapid kinetics. When the second step involves nucleic acid hybridization, the rate of hybridization is optimized by the use of low sequence complexity of the oligonucleotide/complementary oligonucleotide (16 to 50 basepairs) and the possibility of multiple sites for hybridization of a single polymer to the solid support. Figure 1 summarizes the concept in a diagrammatic form.

The polymers do not play a role in the nucleic acid hybridization other than as a support for the capture probes. Their chemical composition is a non-critical factor so long as the polymers are able to support the capture polynucleotides and do not inhibit the hybridization reaction. Two types of polymers are of preferred use: water soluble polymers and microbeads.

### 1. Water Soluble Polymers

Water soluble polymers are defined by their ability to be solvated and are miscible with water or buffered aqueous solutions over all useable concentrations. Water soluble polymers may be homo or heteropolymers and include polymers such as the polyoxides, polyethers, polyallylamine, poly(ethylene imine), acrylic group polymers, vinyl group polymers, polysaccharides, modified starches, methyl-and hydroxypropyl methyl celluloses, hydroxyethylcellulose, carboxymethylcellulose, the polyvinyl alcohols, the ethylene oxide polymer group, polypeptides, polynucleotides, and the polyvinylpyrrolidines, or any other water soluble biopolymer. Poly(ethylene imine), polyvinylamine, polyallylamine and linked oligo- or polynucleotides are preferred. The size range of the water soluble polymers is from about 600 to 100,000 daltons. The preferred range is from about 10,000 to about 70,000 daltons.

The units of the water soluble polymers are typically joined together by direct polymerization process bonding or by coupling agent linking. Direct polymerization produces interbonding of available chemical groups or backbone moieties in adjacent units. For example, oxidative enzymes can be used to polymerize monomer units by oxidative crosslinking. Alternatively, a coupling agent, derived from a bi-functional or multifunctional organic cross-linking agent, bonds with the appropriate chemical group or backbone moiety of the units. In this context the term "coupling agent" denotes the linkage group after bonding and the term cross-linking reagent denotes the linkage compound before bonding.

A preferred cross-linking reagent has generic formula I: 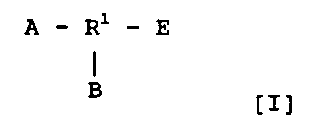 Reactive groups A, B and E of formula I are independently selected from hydrogen, a carboxylic acid group, an acid halide, an activated ester, a mixed anhydride, an iminoester, a primary amine, an aldehyde group, an α-halo acylcarbonyl group, a hydrazine group, an acyl hydrazide group, an azide group or an N-maleimide group. At least two of A, B and E are other than hydrogen. Multifunctional cross-linking reagents, with more than three reactive groups which are similar to A, B, and E are also within the scope of the invention. These additional reactive groups will be independently selected from the foregoing definitions of A, B, and E.

R¹ of formula I is an aliphatic group of at least two carbons and typically no more than 10 carbons, a cycloalkane group of 5-10 carbons, or an aromatic group of at least six carbons.

Choice of the reactive groups of formula I will depend upon the selection of the chemical groups or backbone moieties of the units in the polymer which are to be linked. Each kind of chemical group or backbone moiety will react with its corresponding appropriate reactive group or groups of formula I. An amine group will react with a carboxylic acid group, an acid halide, an activated ester, a mixed anhydride, an acyl imidazole, an N-(carbonyloxy)imide group, an iminoester, an azide or an aldehyde group. An oxidized 1,2-diol group (a dialdehyde) will react with a primary amine, a hydrazine group, an azide or an acyl hydrazide group. A carboxylic acid group will react with a primary amine, a hydrazide group, an azide or an acyl hydrazide group. A mercaptan group will react with a carboxylic acid group, an acid halide, an activated ester, a mixed anhydride, an acyl imidazole, an N-(carbonyloxy)imide, an alpha-beta-unsaturated lactone or maleimide. A carbon-hydrogen bond will react with an azide (nitrene). Reaction conditions are well known and preferred examples are provided below. Preferred monomer units would include: -O-(CH-CH₂)-; -(CH-CH₂); -NH-CH₂-CH₂-; -O-(CH₂)-; -C-(CH₂)-; monosaccharides, amino acids, or combinations thereof.

### 2. Microbeads

Alternatively, the target capturing polymers may be microbeads. The microbeads include water insoluble structures both spherical and nonspherical. The microbead composition may be the same or different as for the water soluble polymers. Preferred compositions include polystyrene, polystyrene/latex, silica, magnetic, teflon or other type beads having reactive functionalities on their surfaces. The preferred size is 0.5 to 120 microns. The preferred functionalities are with carboxy, thiol, or amine. In addition, the bead possessing or being coated by the polymer compounds described in (1) above would be included. Magnetic beads are described in U.S. Patent No. 4,672,040 which is incorporated by reference herein.

The surface of the microbeads can be activated by selecting monomers with appropriate moieties or by surfacing the bead with appropriate reagents. The choice of the reactive groups on the surface of any microbead will depend upon the selection of the chemical groups or backbone moieties of the units which are to be linked onto the surface of the microbead.

Each kind of chemical group or backbone moiety will react with its corresponding appropriate reactive group or with groups of formula I. An amine group will react with a carboxylic acid group, an acid halide, an activated ester, a mixed anhydride, an acyl imidazole, an N-(carbonyloxy)imide group, an iminoester, an azide or an aldehyde group. An oxidized 1,2-diol group (a dialdehyde) will react with a primary amine, a hydrazine group, an azide or an acyl hydrazide group. An activated carboxy group will react with a primary amine, a hydrazide group, an azide or an acyl hydrazide group. A mercaptan group will react with a carboxylic acid group, an acid halide, an activated ester, a mixed anhydride, an acyl imidazole or an N-(carbonyloxy)imide. A carbon-hydrogen bond will react with an azide (nitrene).

### B. Assay Formats.

The principle application for this invention is in nucleic acid hybridization. In general, hybridization methods comprise the steps of a) contacting a single stranded target polynucleotide sequence or denatured double strand polynucleotide target with a single stranded polynucleotide probe having adequate base complementarity, and b) detecting the resulting polynucleotide duplex formed between target and probe. Conventional hybridization formats which are particularly useful include those where the sample nucleic acid is contained in a cell (in-situ), those where either the polynucleotide target or the polynucleotide probe is immobilized on a polymer (solid-phase hybridization), and those where all polynucleotide species are in solution (solution hybridization).

The particular hybridization format is not critical to the invention. As improvements in current hybridization formats are made and new formats developed, they can readily be applied to this invention.

Where the chosen format involves a solid support, the solid support may consist of a membrane-type solid support (two dimensional solid support), a bead-type solid support or other solid surfaces such as a microtiter well.

Sequestration of the polymers need not be limited to nucleic acid hybridization. Any ligand/receptor combination may be substituted. These would include antibody/antigen, hormone/receptor, streptavidin/biotin combinations and the like. For sequestration, any ligand/receptor combination will work as long as the rate of sequesterization is slower than the rate of solution hybridization for the target to the oligonucleotides attached to the polymer.

For mixed phase assays using solid supports, the preferred ligand/receptor combination is complementary oligonucleotides. Specifically, the sequences of some or all of the oligonucleotides attached to the polymer are also complementary to a sequence of oligonucleotide covalently attached to the solid support (see Figure 1). Thus, the polymer will form duplexes with both the target nucleic acid and the nucleic acid on the solid support.

Due to diffusional constraints, the polymer hybridizes to the target nucleic acid in solution with much more rapid kinetics than the polymer hybridizes to the solid support. Therefore, the first step in the process is generally the hybridization or capture of the target nucleic acid onto the oligonucleotide immobilized on the polymeric structure. The second step is the translational diffusion of the polymer (with or without captured target nucleic acid) to the solid support. Upon completion of this second hybridization event, the target-containing complex is considered sequestered.

The overall rate of sequestering of the target nucleic acid onto a solid support is greatly increased using this procedure. The rate of hybridization of the target nucleic acid to the oligonucleotide on the polymer occurs with nearly ideal solution kinetics since the polymer is uniformly distributed in the solution and can be present at a concentration such that the capture oligonucleotide drives the reaction with pseudo-first order kinetics. The hybridization and sequestration of the polymer onto the solid support requires translational diffusion of the structure and, hence, the solution is preferably agitated in some manner to promote the translational diffusion. Once the polymer encounters the solid support, the sequestration is very rapid since only low sequence complexity hybridization occurs between two complementary oligonucleotides.

The polymers are covalently conjugated to either a single species or multiple species (having different nucleic acid base sequences) of oligonucleotides via the chemistries described below. The desired polymers have more than two and typically less than 1,000 oligonucleotides attached to each polymer. The sequence of the oligonucleotide attached to the polymer is complementary to a target nucleic acid (e.g., rRNA, RNA, denatured plasmid DNA, denatured DNA, etc.).

It is also possible for the polymer to make multiple attachments with the oligonucleotides on the solid support and also on the target if more than one type of oligonucleotide is present on the polymer, and, therefore, the complex can be made more stable than a target nucleic acid directly held to a solid support or target via a single oligonucleotide attachment.

An alternative format combines the microbeads (water insoluble polymers) with the water soluble polymer. Where one combines water soluble polymers and beads, both may act as an intermediate in the capture of target nucleic acid to the solid support. Preferably, the water soluble polymer captures the target as previously described, then this complex attaches to the microbead. Finally, this ternary complex is captured by the solid support. Additional combinations of large and small beads and polymers are also operable. Such combinations would represent ternary or quaternary complexes prior to sequestering to a solid support.

For solid phase hybridizations, a polynucleotide species is immobilized onto the solid phase. The polynucleotide can either be covalently or non-covalently bound to the solid phase. Noncovalent supports include such polymers as nitrocellulose, derivatized nylon (i.e. Nytran (TM)), and fluorinated polyhydrocarbons. Covalent binding supports comprise materials that have reactive moieties on their surfaces that can serve as sites of attachment of an appropriately activated polynucleotide.

The solid support can be two dimensional such as a membrane, or a three dimensional surface such as magnetic or plastic beads, or the reaction vessel such as the walls of a microtiter well.

A preferred form of a solid phase hybridization is the sandwich assay. In this method a polynucleotide sequence (capture sequence) complementary to a given sequence on the target polynucleotide species is immobilized on a solid support. The immobilized polynucleotide sequence is contacted with the target sequence in the presence of a labeled polynucleotide sequence (signal sequence) complementary to a discreet sequence on the target polynucleotide species (one step sandwich assay) or after capture of the target sequence a subsequent hybridization with the signal sequence (two step sandwich assay) is performed. In the performance of the sandwich assay, one can use a first set of capture beads to bind the target to a second set of beads, a membrane, a dipstick, or a wall of a mictotiter well.

### C. Hybridization Conditions.

The particular hybridization technique is not essential to the invention. Hybridization techniques are generally described in Nucleic Acid Hybridization, A Practical Approach, Ed. Hames, B. D. and Higgins, S. J., IRL Press, 1985. As improvements are made in hybridization techniques, they can readily be applied.

The specific hybridization conditions are not critical and will vary in accordance with the investigators preferences and needs. Various hybridization solutions may be employed, comprising from about 20 to 60% volume, preferably 40%, of a polar organic solvent. A common hybridization solution employs about 30-60% v/v formamide, about 0.05 to 1M sodium chloride, about 0.01 to 0.1M buffers, such as sodium citrate, Tris HCl, PIPES, HEPES, EPPS, about 0.05 to 0.5% detergent, such as sodium dodecylsulfate, and between 1-10 mM EDTA, 0.01 to 5% ficoll (about 300 - 500 kilodaltons), 0.1 to 5% polyvinylpyrrolidone (about 250-500 kdal), and 0.01 to 10% bovine serum albumin. Also included in the typical hybridization solution will be unlabeled carrier nucleic acids from about 0.1 to 5 mg/mL, e.g., partially fragmented calf thymus or salmon sperm, DNA, and/or partially fragmented yeast RNA and optionally from about 0.5 to 2% w/v glycine. Other additives may also be included, such as volume exclusion agents which includes dextran sulfate and polystyrene sulfonic acid.

Various degrees of stringency of hybridization can be employed. As the conditions for hybridization become more stringent, there must be a greater degree of complementarity between the probe and the target for the formation of a stable duplex. The degree of stringency can be controlled by the temperature, ionic strength, the inclusion of polar organic solvents, and the like. For example, temperatures employed will normally be in the range of about 20° to 90°C, usually 25 to 75°C. The stringency of hybridization is also conveniently varied by changing the ionic strength and polarity of the hybridization solution through manipulation of the concentration of formamide within the range of 20 to 50%.

After hybridization at a temperature and time period appropriate for the particular hybridization solution used, the glass, plastic, or filter support to which the probe-target hybrid is attached is introduced into a wash solution typically containing similar reagents as provided in the hybridization solution. These reagents may be at similar concentrations as the hybridization medium, but often they are at lower concentrations when more stringent washing conditions are desired. The time period for which the support is maintained in the wash solutions may vary from minutes to several hours or more.

Either the hybridization or the wash medium can be stringent. After appropriate stringent washing, the correct hybridization complex may now be detected in accordance with the nature of the label.

### D. Labeling and Detection.

Various labels can be used in hybridization assays benefiting from this invention. Labels act as reporter groups for detecting duplex formation between the target sequence and their complementary signal sequence. A reporter group as used herein is a group which has a physical or chemical characteristic which can be measured or detected. Detectability may be provided by such characteristics as color change, luminescence, fluorescence, or radioactivity; or it may be provided by the ability of the reporter group to serve as a ligand recognition site.

Probes may be labelled by any one of several methods typically used in the art. A common method of detection is the use of autoradiography with ³H, ¹²⁵I, ³⁵S, ¹⁴C, or ³²P labelled probes or the like. Other reporter groups include ligands which bind to antibodies labeled with fluorophores, chemiluminescent agents, and enzymes. Alternatively, probes can be conjugated directly with labels such as fluorophores, chemiluminescent agents, enzymes and enzyme substrates. Alternatively, the same components may be indirectly bonded through a ligand- antiligand complex, such as antibodies reactive with a ligand conjugated with label. The choice of label depends on sensitivity required, ease of conjugation with the probe, stability requirements, and available instrumentation.

The choice of label dictates the manner in which the label is incorporated into the probe. Radioactive probes are typically made using commercially available nucleotides containing the desired radioactive isotope. The radioactive nucleotides can be incorporated into probes, for example, by using nick-translation, by tailing of radioactive bases to the 3' end of probes with terminal transferase, by copying M13 plasmids having specific inserts with the Klenow fragment of DNA polymerase in the presence of radioactive dNTP's, or by transcribing RNA from templates using RNA polymerase in the presence of radioactive rNTP's.

Non-isotopic probes can be labeled directly with signal (e.g., fluorophore, chemiluminescent agent, or enzyme) or labeled indirectly by conjugation with a ligand. For example, a ligand molecule is covalently bound to the probe. This ligand then binds to a receptor molecule which is either inherently detectable or covalently bound to a detectable signal, such as an enzyme or photoreactive compound. Ligands and antiligands (receptors) may be varied widely. Where a ligand has a natural "antiligand," namely ligands such as biotin, thyroxine, and cortisol, it can be used in conjunction with its labeled, naturally occurring antiligand. Alternatively, any haptenic or antigenic compound can be used in combination with a suitably labeled antibody.

Enzymes of interest as reporter groups will primarily be hydrolases, particularly phosphatases, esterases, ureases, and glycosidases, or oxidoreductases, particularly peroxidases. Fluorescent compounds include fluorescein and its derivatives, rhodamine and its derivatives, dansyl, umbelliferone, etc. Chemiluminescers include luciferin, luminol, and 1,2 dioxetanes.

The amount of labelled probe which is present in the hybridization solution may vary widely. Generally, substantial molar excesses of probe over the amount of the target nucleic acid will be employed to enhance the rate of binding of the probe to the target DNA.

For example, where the label is radioactive, the support surface with associated hybridization complex substrate is exposed to X-ray film. Where the label is fluorescent, the sample is detected by first irradiating it with light of a particular wavelength. The sample absorbs this light and then emits light of a different wavelength which is detected. Where the label is an enzyme, the sample is detected by incubation with an appropriate substrate for the enzyme. The signal generated may be a colored precipitate, a colored or fluorescent soluble material, or photons generated by bioluminescence or chemiluminescence.

Detection of a hybridization complex may require the binding of a signal generating complex to a duplex of target and probe polynucleotides or nucleic acids. Typically, such binding occurs through ligand and antiligand interactions as between a ligand-conjugated probe and an antiligand conjugated with signal.

The label may also allow indirect detection of the hybridization complex. For example, where the label is a hapten or antigen, the sample can be detected by using antibodies. In these systems, a signal is generated by attaching fluorescent of enzyme molecules to the antibodies or in some cases, by attachment to radioactive label.

### E. Conjugation of Capture Polynucleotides to the Polymers.

### 1. Functionalization of the Capture Oligonucleotide.

Capture polynucleotides may be created and linked to any of the above described polymers according to the following chemistries.

DNA probes are preferably chemically synthesized using commercially available methods and equipment. For example, the solid phase phosphoramidite method can be used to produce short probes of between 15 and 50 bases and have a molecular weight of less than 16,000 daltons. These polynucleotides are referred to herein as "short probes or oligonucleotides." (Caruthers, et al., Cold Spring Harbour Symp. Quant. Biol., 47:411-418, 1982, and Adams, et al., J. Am. Chem. Soc., 105:661, 1983).

When synthesizing a probe for a specific target, the choice of nucleotide sequence will determine the specificity of the test. For example, by comparing DNA sequences from several virus isolates, one can select a sequence for virus detection that is either type specific or genus specific. Comparisons of DNA regions and sequences can be achieved using commercially available computer programs.

The preferred capture nucleic acids for use in this invention are synthetic oligonucleotides of between 20 and 100 bases. During synthesis a linker arm containing a blocked amine group can be coupled using conventional chemistry to the 5'-hydroxyl group of an oligonucleotide. The activated oligonucleotides used as starting materials for this invention can be derived through several methods. The reagents for the attachment of primary linker arms terminating in a primary amine are commercially available. A primary amine is the preferred group for attachment to the heterobifunctional reagent, and its attachment via a hexyl arm is preferred. Starting materials suitable for use in this invention are described in PCT U.S. 86/01290; Nucl. Acids Res., 15:3131 (1987); Nucl. Acids Res., 15:2891 (1987); and Nucl. Acids Res., 14:7985 (1986).

Specifically, an oligonucleotide possessing a 5'-terminal structure such as 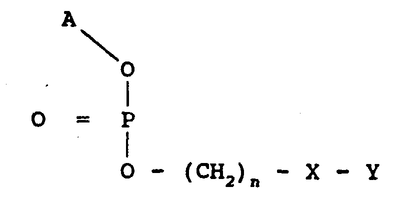 is employed where A is a poly or oligonucleotide, and n and m are between 2 and 12, inclusive; X is an NH or NHC:O(CH₂)ₘNH, and Y is a thiol reactive moiety. The oligonucleotide can range between about 9 and 50 bases, preferably between about 15-30 bases, with only the 5'-hydroxyl requiring modification for attachment. The preferred thiol reactive moieties are an alpha-halo-acyl, an alpha- or beta-unsaturated carbonyl, or an alpha- or beta-unsaturated lactones. The attachment of thiol reactive substituents upon the primary amines has been described in detail in International Patent Application WO 89/06701, published July 27, 1989 (see, in particular, pages 4-8), all of which is incorporated by reference herein. The most preferred thiol reactive moieties are alpha-haloacetamidobenzoyl and 4-(N-maleimidomethyl) cyclohexane-1-carbonyl. Preferred halogens are iodine and bromine. The thiol-reactive oligonucleotides are referred to as activated oligonucleotides.

Alternatively, an oligonucleotide can be modified at its 3'end with a linker arm containing a blocked amine group. This can be accomplished by conducting DNA synthesis on a solid support containing a conjugated ribonucleotide. After removal from the solid support, a DNA oligonucleotide is obtained which contains a single 3'-terminal ribonucleotide. This can be modified with a linker arm containing a nucleophilic amine by, for example, 1) oxidizing the ribonucleotide cis-glycol with periodate, 2) treating oligonucleotide so modified with, for example, butane diamine to form a Schiff base, and 3) treating with sodium borohydride or cyanoborohydride to form a stable reduced Schiff base derivative in which one of the amines is left free for subsequent conjugation. "Practice and Theory of Enzyme Immunoassays," P. Tijssen at page 237, Elsevier Science Publishers B.V. Amsterdam, Netherlands.

DNA bases can also be modified to become thiol reactive before or after preparation in a DNA synthesizer. Using the post-synthesis approach, natural nucleic acids (DNA and RNA) and molecularly cloned nucleic acids, as well as synthetic nucleic acids, can be modified. In the case of cytosine, a more nucleophilic amine is linked to the 4-position of cytosine by a variety of chemistries. The amine can be added by treatment with hydrazine to generate N-4-aminocytosine (Sverdlov, E.D., et al., FEBS Letters, 62, p. 212., Feb. 1976). This reaction is catalyzed by bisulfite. Alternatively, an amine can be added to the 4-position by bisulfite catalyzed transamination reactions where a diaminoalkane is added to the 4-position (Shapiro and Weisgras, Biochem. and Biophys. Res. Comm., 40:839, 1970).

Bi-functional semicarbazide can also be used to add nucleophilic amines to the 4-position of cytosine (Hayatsu and Ukita, Biochemical and Biochemical Research Communications, 14:198, 1964). The base with the free amino group in the oligo is then made thiol reactive through the reaction of the nucleophilic amine and a N-hydroxysuccinimidyl (NHS) ester of a carboxylic acid derivative substituted with a alpha- or beta-unsaturated carbonyl, or alpha-halocarbonyl groups or the like.

Other nucleic acid bases with nucleophilic amines on other positions can be used for reactions with NHS esters of a carboxylic acid derivative substituted with a alpha- or beta-unsaturated carbonyl, or alpha-halo-acyls. For instance, 5-[N-(7-aminohexyl)-1- acrylamido]-2'-deoxyuridine 5'-triphosphate (Calbiochem, La Jolla, CA) can be added to the 5' end of oligonucleotides in reactions with terminal deoxnucleotidyl transferase ("Molecular Cloning, A Laboratory Manual," T. Maniatis, et al. eds., Cold Spring Harbor Laboratory, p. 148, 1982).

The preferred method for chemical immobilization of the oligonucleotide is accomplished by the following procedure. The oligonucleotide bearing the tethered amine is covalently bound to a reagent having two different types of functional groups. These bifunctional reagents typically have an N-hydroxysuccinimidyl [NHS] ester, which is amine reactive as the first functional group, and a thiol reactive group, as the second functional group. The NHS ester acylates the free amine of the 5' end of the oligonucleotide. The acylating conditions are well known and highly selective. The exocyclic amines on adenine and cytosine bases are not nucleophilic and do not readily react with the NHS ester. After acylation to the oligonucleotide, the thiol reactive functional group is available for conjugation with surfaces containing free sulfhydryl groups.

An oligonucleotide with a tethered nucleophilic amine linked to its 5' end can be derivatized with such thiol-reactive groups by relatively simple chemistry. For example a reagent such as N-Succinimidyl (4-iodoacetyl) aminobenzoate [SIAB] is added to the oligonucleotide in aqueous solutions at a 10 to 100 fold molar excess. The pH is buffered between about 6 and 9 and the temperature is between about 20° and 30°C. After a reaction period of about 30 to 90 minutes the excess unreacted reagent is removed by exclusion gel filtration.

The preferred thiol reactive moiety is an alpha-halo-acyl compound or an alpha- or beta-unsaturated carbonyl compound. The thiol reactive group may include: maleimides, pyridyl sulfides, and active halogens, alpha- or beta-unsaturated lactones and the like. The active halogens are typically alpha-haloacyl. Useful halogens include chlorine, bromine iodine, and fluorine with iodine and bromine being preferred. Reagents useful for this invention can be purchased from Pierce Chemical Co., Rockford, IL. Examples include: N-Succinimidyl 4-(iodoacetamido)benzoate (SIAB), and Sulfosuccinimidyl (4-iodoacetamido)benzoate (Sulfo-SIAB).

### 2. Functionalizing of a Polymer.

The surfaces of the polymers of this invention are preferably modified with sulfhydryl groups for subsequent reaction with the thiol-reactive bound oligonucleotide described below. The preferred method is to modify the polymer or surface with disulfide derivatives containing nucleophilic amines which participate in conjugation, and then to reduce the disulfide groups to monothiols by treatment with, for example, dithiothreitol, in order for the thiol reactive portion of the activated oligonucleotide to covalently bind to the surface.

Several types of polymers are available for modification. Preferred water soluble polymers include polymers such as the polyoxides, polyethers, poly(ethylene imine), acrylic group polymers, vinyl group polymers and the polyvinylpyrrolidines. Poly(ethylene imine), polyallylamine and polyvinylamine are most preferred. The surfaces of the following beads may be modified: sephadex, agarose, polystyrene beads, teflon beads, polystyrene/latex beads, latex beads or any microsphere or bead possessing an activated carboxylate, sulfonate, phosphate or similar activatable group.

The polymers may comprise a reactive surface of the above materials and a substratum. Although the need for reactive moieties to covalently bind the capture polynucleotides restricts the choice of surface, the substratum may be the same as the surface or be different. Where different, the substratum can be virtually any inert composition.

For example, silica particles which are either porous or non-porous and possessing pre-activated surfaces or amine type moieties may be used. These silica particles may be obtained commercially.

The following chemical moieties can be attached to the polymer surface: any aminoalkyl or aryl disulfides (for coupling of the oligonucleotides), any mercaptans (for surface modification), any amino thiol alcohols (for coupling of oligonucleotides), and aryl disulfides, amino aryl disulfides, or aminoalkylaryl disulfides (for coupling of the oligonucleotides).

The organic residues are non-critical features of this invention. There is virtually no limit to the substitutions which could be made so long as the ability to retain the thiol ether or thiol linkage is preserved.

In the case of spheres or beads consisting of polystyrene, polystyrene/latex, latex, or any other polymer possessing an activatable carboxy group, the procedure of J.V. Staros et al., Analytical Biochemistry 156:220-222 (1986) is employed. The beads are placed in a 100,000 to 1,000,000 molar excess of 1-ethyl-3-(3-diethylaminopropyl)carbodiimide (EDC), or other carboxyl activating reagent, in aqueous buffer at pH 5.0 to 5.5 for 2 minutes at 20° to 30° C. The excess and unreacted EDC is then removed by centrifugation or filtration of the beads. The activated beads are exposed to 0.01 to 1 molar solution of an aminoalkyldisulfide or an aminoalkylmercaptan preferably cystamine or cysteamine (2-aminoethanethiol) in a non-amine containing buffer such as sodium borate, sodium phosphate or sodium carbonate at about pH 7 to 9 and allowed to react for 1 to 24 hours.

After appropriate washing steps, followed by centrifugation or filtration, the disulfide of the conjugated cystamine is reduced to a sulfhydryl using a reducing agent such as 0.01 to 0.1 M dithiothreitol or dithioerythritol If cysteamine was employed in the last step, the beads do not require a reduction step.

### 3. Conjugation and Optional Post-Conjugation Modification of the Activated Oligonucleotide to the Thiolated Polymer Surface.

The thiolated surface of the polymer, is reacted with the activated oligonucleotide, using a 2 to 100-fold excess of activated oligonucleotide over the maximum oligonucleotide binding capacity of the polymeric structure.

After conjugation of the surface of the polymeric structure with a capture DNA probe, there may be a large excess of free sulfhydryl groups. The ratio of these remaining sulfhydryl groups to covalently coupled capture oligonucleotide can range from 10:1 to 1,000,000:1. The presence of these excess thiol groups permits a second modification of the surface of the polymeric structure by the selective chemical coupling of R-SH groups, where R is any chemical functionality. This chemical coupling can be either irreversible (e.g., by reaction with a maliemide derivative) or reversible (e.g., by formation of a disulfide bond).

The ability to conduct a second modification of the surface of the polymeric structure is important for achieving a variety of desired surface properties on the polymeric structure. The following are illustrative of some possible examples:
a. The thiolated surface provides the chemical means for irreversible modification in a second conjugation step by treatment of the free bonds. Such sulfhydryl conjugations can modify the charge, hydrophobicity, hydrophilicity, color, reflectance, transmittance, porosity, frictional coefficient, porosity, conductivity and heat capacity of the surface. The thiolated surface also provides the means whereby proteins, nucleic acids, antibodies, antigens or other macromolecules of interest may be irreversibly coupled to the polymeric structure. Such modified surfaces will control, in part, the rate of hybridization of target nucleic acid to the capture probe on the solid surface support and will be important in reducing the level of nonspecific background arising during the detection of the hybridized labelled probe.
b. In contrast to the irreversible modification described directly above, the thiolated surface also provides the chemical means for reversible modification in the second conjugation step by the formation of disulfide bonds between the surface and a desired reagent (e.g., formation of surface-S-S-R bonds). Such conjugations can modify transmittance, frictional coefficient, porosity, conductivity and heat capacity of the surface. The thiolated surface also provides the means whereby specific proteins, nucleic acids, antibodies, antigens or other macromolecules of interest may be reversibly coupled to the polymeric structure. These types of moieties may serve as receptors, ligands, or substrates in the signal development processes.

The following examples are offered by way of illustration and not by way of limitation.

### EXAMPLES

Example 1 provides general methods and materials.

In Examples 2, 3 and 4 the rate and extent of specific sequesterization of target nucleic acid with and without the use of a target capturing polymer is compared. The results indicate that the sensitivity (extent) of sequesterization is increased 5 to 20-fold and that the rate of sequestration is increased 10 to 25-fold.

### Example 1. General Methods and Materials.

### Solutions and Buffers.

Filtered wash (FW) is 0.09 M sodium chloride, 50 mM Tris- HCl pH 7.6, 25 mM EDTA; SDS/FW is FW and 0.1% Sodium dodecyl sulfate (SDS); horseradish peroxidase (HRP) substrate solution is 0.1 M sodium citrate pH 5.5, 0.5 mg/ml 4-methoxy-1-naphthol, 0.02 mg/ml 3-methyl-2-benzo-thiazolinone hydrazone and 0.0135% hydrogen peroxide; alkaline phosphatase (AP) substrate solution is 1 mM 5-bromo-4-chloroindoyl-3-phosphate, 1 mM nitroBlue tetrazolium, and 0.01% Tween 20 in TMNZ which is 100 mM Tris.HCl pH 8.5, 100 mM NaCl, 50 mM MgCl₂, and .1 mM ZnCl₂; lysis solution is 3 M guanidinium thiocyanate, 2% N-lauroylsarcosine (sarcosyl), 50 mM Tris-HCl pH 7.6, 25 mM EDTA.

Materials:
a. Oligonucleotides.
   Two oligonucleotides complementary to two different regions of the 16S-ribosomal RNA of Bacteroides gingivalis were synthesized on an Applied Biosystems DNA synthesizer Model 380B. The sequences for all the oligonucleotides described below are described in Table 1.
b. Poly(ethyleimine) was purchased from Polysciences (Warrington, PA).

### Preparation of Iodoacetamidobenzoylated Oligonucleotides:

10 to 1000 µg of 5'-terminal amine linked oligonucleotide (UP9A) or the 5'-terminal amine complementary (denoted by "C") oligonucleotide UP9A"C") were reacted with an excess of N-succinimidyl 4-(iodoacetamido)-benzoate (SIAB) in an alkaline (pH 8.0 preferably) buffer at 18° to 25° C for 30 to 120 minutes. The unreacted SIAB is removed by size exclusion chromatography on a NAP 25 (Pharmacia) column.

### Preparation of the Solid Support:

A 16 cm² piece of Nytran was incubated with 10 ml of 5 mg/ml iminothiolane in 0.1 M sodium borate at pH 8.3. Incubation is for 30 minutes at ambient temperature. The membrane was washed with 5 changes of the sodium borate buffer described above and the presence and quantification of introduced sulfhydryl groups was determined using 5,5-dithio-bis(2-nitrobenzoic acid). The derivatized membrane was then cut into 0.28 cm² discs and washed once with 0.1 M sodium borate buffer. IAB-oligonucleotide was prepared as described above and mixed with the membrane discs. 300 membrane discs were submerged in 2 ml of 0.1 M sodium borate buffer containing 1.0 mg of IAB-oligonucleotide and the reaction was allowed to proceed at room temperature with constant agitation for 16 hours in the dark.

The discs were then washed sequentially with 0.1 M sodium borate and SDS/FW. 0.1 to 1.2 micrograms of oligonucleotide was bound per filter disc. The unreacted sulfhydryl groups were then capped by treating the filters with 50 mg/ml iodoacetamide in 0.1 M sodium borate pH at 8.3. The filters were then washed further with sodium borate and SDS/FW.

### Preparation of the Poly(ethylene imine)-oligonucleotide polymer:

a. Thiolation: 50 µl of a 30% aqueous solution of poly(ethylene imine) ("PEI" from Polysciences, Warrington, PA), molecular weight of 10,000 was added to 250 microliters of 0.1 M Na borate at pH 8.3 containing 50 mg of iminothiolane and mixed for 1 hour at ambient temperature. The thiolated polymer was then purified by exclusion chromotography on G-25 Sephadex in Na borate buffer at pH 8.3 and the number of thiol groups introduced was determined by titration with 5,5'-dithio-bis(2-nitrobenzoic acid).
b. 500 to 5000 micrograms of Bg1 oligonucleotide was derived with the heterobifunctional cross-linking reagent SIAB as described above (see table 1). The purified oligonucleotide was concentrated using butanol and then taken to near dryness under a vacuum to remove any remaining butanol. 100 to 200 µl of N-methyl-pyrrolidone was added to the oligonucleotide and then 5 µl of the thiolated polymer was added to the oligonucleotide solution and the solution was rigorously mixed for 1 to 18 hours. The conjugate was purified by HPLC exclusion chromatography using a GF-250 column (DuPont). The polymer was concentrated and stored at -20° C in 0.01 M Tris/0.01 M EDTA/0.2% (V/V) mercaptoethanol, and 50% glycerol. Gel analysis indicated than 5 to 15 oligonucleotides were bound per polymer.

### Example 2. Amplification of capture of Bg rRNA using a PEI-oligonucleotide conjugate in a colorimetric assay.

This example employs the use of a 10,000 MW PEI polymer conjugated to Bg1-oligonucleotide which will capture 16s Bg rRNA and hybridize to a nytran solid support which possesses covalently immobilized BglC-oligonucleotide.

3 M GnSCN lysis solution was used to lyse 1 x 10⁸ cells of Actinobacillus actinomycetemcomitans (Aa), Bacteriodes gingivalis (Bg) Bacteriodes intermedius (Bi), Eikenella corrodens (Ec), Fusobacterium nucleatum (Fn), Wolinella recta (Wr) in 100 microliter volumes at 19°C. The samples were then heated to 65°C for 5 to 10 minutes. Biotinylated 24-mer oligonucleotide probes, complementary to conserved regions of bacterial 16s rRNA (signal probes), were added to a final concentration of 100 nanograms per ml.

To one solution, Bg1-10K PEI conjugate was added to a final concentration of 1 microgram/ml (based on oligonucleotide concentration). 5-fold serial dilutions of the lysates were made using diluents containing the biotinylated signal oligos and with or with or without the PEI-oligonucleotide conjugate and 1 x 10⁸ total cells of Aa, Bi, Ek, Fn and Wr. The solutions were then incubated for 10 minutes at ambient temperature with nytran discs, upon which 1 µg of Bg1C or Bg1 specific oligonucleotide probe (capture probe) was covalently immobilized. During the incubation, the containers were mildly agitated using a rotary platform shaker.

The solid supports were then washed with SDS/FW at ambient temperature and then incubated with 10ng/ml of Strepavidin/Horseradish peroxidase (SA/HRP) conjugate in SDS/FW for 5 minutes at ambient temperature. The solid supports were then washed with SDS/FW, FW, and then the presence of peroxidase was determined by incubating the filter with the HRP substrate solution described in Example 1 to form an insoluble product. The results indicated that in the 10 minute hybridization 3 x l0⁵ cells were detected using the capture amplification polymer whereas without the amplification polymer, 8 x 106 were detected. Background levels were not increased with the use of the amplification polymer. The control in which Aa, Bi, Ek and Wr cells were present and Bg was absent showed no color indicating that the capture of Bg was specific.

### Example 3. Determination of the rate and extent of enhanced capture of target nucleic acid using a target capturing polymer as an intermediate in the sequesterization of target nucleic on a solid support.

This example describes the rate and extent of sequesterization of target nucleic acid (16s rRNA) onto a solid support with and without the use of a polymer/oligonucleotide conjugate as an intermediate in the hybridization procedure.

The experiment employed the use of a 70,000 MW PEI polymer conjugated to UP9a-oligonucleotide (a "universal" oligonucleotide probe for 16s bacterial rRNA) which will capture 16s Bg rRNA and hybridize to a nytran solid support which possesses covalently immobilized UP9A"C"-oligonucleotide.

3 M GnSCN lysis solution was used to lyse 1 x 10⁷ cells of Bg in 100 microliter volumes at 19°C and heated at 65°C for 5-10 minutes. A ³²P 24-mer oligonucleotide probe (specific activity of 5 x 10⁷ cpm/microgram) complementary to hypervariable regions of bacterial 16s rRNA (signal probes) were added to a final concentration of 10 nanograms per ml. To one series, a solution UP9a-70K PEI conjugate was added to a final concentration of 1 microgram/ml (based on oligonucleotide concentration). For each kinetic curve, seven duplicate time points were employed. The solutions were then incubated with nytran discs which had covalently immobilized 1 microgram of UP9a or UP9aC specific oligonucleotide probe (capture probe) for 0, 5, 10, 15, 30, 45 or 60 minutes at ambient temperature. During incubation, the samples were mildly agitated as previously described. The solid supports were then washed 5 times with SDS/FW at ambient temperature and bound radioactivity was monitored by scintillation counting.

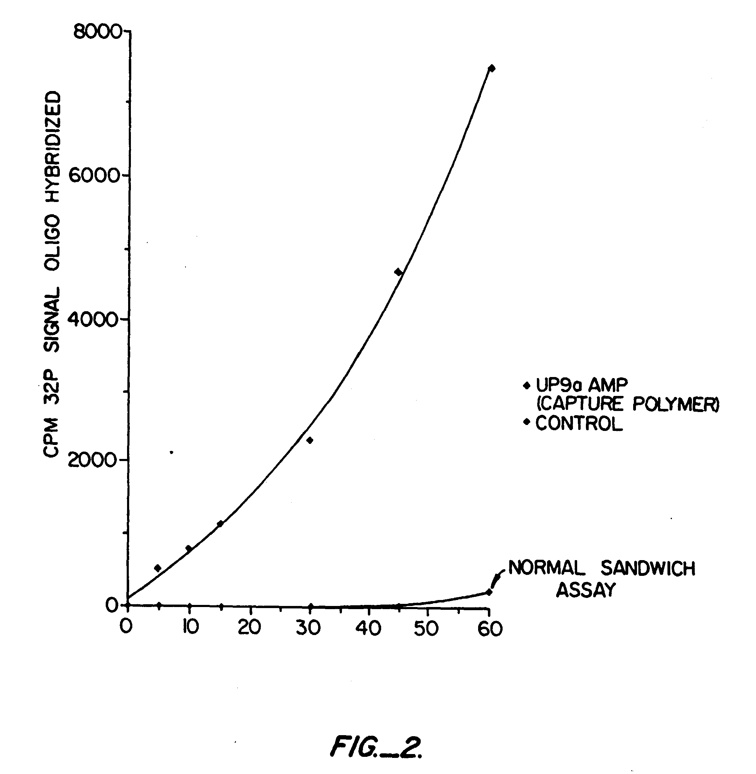

The results are shown in Figure 2. A comparison of the two kinetic profiles with and without the use of the polymer indicate that the rate of the reaction containing the intermediate polymer is increased 10 to 25 fold and the extent of the reaction at the 60 minute time period is increased approximately 20-fold. Thus, in 5 minutes about twice the level of sequesterization of the target nucleic acid is achieved using the PEI/oligonucleotide conjugate as is achieved in 1 hour without the use of a target capturing polymer as an intermediate.

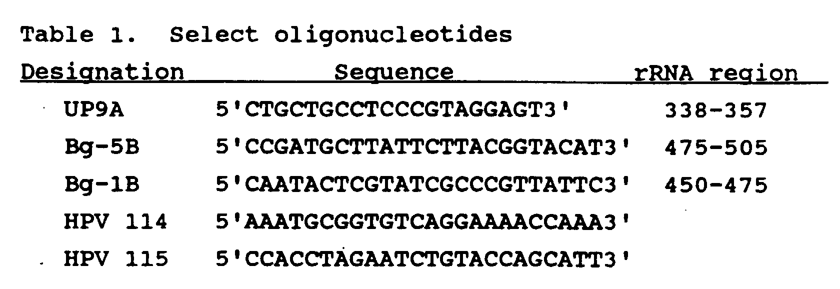

### Example 4: Amplification of capture of denatured double strand DNA using a capture polymer complementary to one strand of the target double-strand DNA.

This example employs the use 70,000 MW PEI polymer possessing 24-mer oligonucleotides complementary to an human papilloma virus sequence contained on plasmid DNA to increase the rate and extent of capture of denatured double strand DNA onto a solid support.

The sequences for this example are described in Table 1. Nytran filters containing 0.29 micrograms of HPV 115"C" oligonucleotide ("C" denotes the complementary sequence of HPV 115 oligonucleotide described in table 1), 50, 75, or 100 nanograms of HPV 115-70K PEI polymer, 1 x 10⁹ copies of PBR322 plasmid containing the HPV serotype 16 genome (DNA which is complementary to various (HPV) genomes were prepared from recombinant inserts in a pBluescript cloning vector (i.e. pHPV-16 is a genomic sequence of human papilloma virus type 16 inserted into the BamH I restriction site of pBluescript II\). pBluescript II\ is a 2.95 kb colony producing phagemid derived by replacing the pUC19 polylinker of pBS (+/-) with a synthetic polylinker containing 21 unique restriction sites) which was end labelled with P32-dATP using T4 kinase, all of which was contained in 0.6 M NaCl, 90 mM Tris pH = 8.0, 10 mM EDTA, 0.5% SDS, 30% deionized formamide (hybridization solution). The solution (100 microliters total volume) was then heated in a water bath to 90 degrees C for 5 minutes. The solution was then incubated at 20 degrees C for 4 hours at which time the hybridization reaction was stopped and the solid support (the filter) removed from the solution.

The filters were then washed twice with the hybridization solution described above and then three times with SDS/FW. The filters were then monitored for radioactivity using liquid scintillation counting. Table 2 describes the extent of capture of the llkb HPV 16 containing plasmid.

**Table 2:**

| POLYMER CONCENTRATION | CPM CAPTURED | %CAPTURE¹ |
|---|---|---|
| no polymer | 1014 | 3% |
| 50 ng | 33087 | 86% |
| 75 ng | 24147 | 63% |
| 100 ng | 28939 | 75% |

| | | |
|---|---|---|
| 1) The cpm of total availible target in each reaction was 38,000 cpm. It was assumed only 1/2 of the total input cpm was availible for capture since the polymer possessed only one oligonucleotide complementary to the sense strand. | | |

Therefore it was demonstrated that the use of the capture polymer increased the sequestering the of llkb denatured plasmid DNA by an average of 25-fold over direct capture of the DNA by the solid support.

## Claims

1. A method for enhanced capture of a target polynucleotide comprising:
(a) contacting the target polynucleotide with a complementary capture polynucleotide covalently bound to a polymer,
wherein the polymer
(1) is a water soluble polymer or a microbead coated with a water soluble polymer;
(2) has at least two copies of the capture polynucleotide covalently bound thereto; and
(3) has mobility in solution;
(b) permitting hybridization to occur between the target polynucleotide and the capture polynucleotide, wherein mobility of the polymer in solution amplifies hybridization; and
(c) binding the polymer to a solid surface.

2. The method of Claim 1 wherein the hybridization with polymer amplification is increased about 10-25 fold during a 10 minute hybridization, as compared to hybridization without polymer.

3. The method of Claim 1 wherein the polymer is selected from the group consisting of polyoxides, polyethers, poly(ethylene imine), acrylic group polymers, vinyl group polymers, polyallyl polymers, polysaccharides, polynucleotides, polypeptides and polyvinylpyrrolidine polymers.

4. The method of Claim 1 wherein the water soluble polymer has a molecular weight of between about 600 and about 100,000 daltons.

5. A method for enhanced capture of a target polynucleotide comprising:
(a) contacting the target polynucleotide with a complementary capture polynucleotide covalently bound to a polymer,
wherein the polymer
(1) is a water soluble polymer or a microbead coated with a water soluble polymer;
(2) has at least two copies of the capture polynucleotide covalently bound thereto;
(3) has a ligand member of a ligand/receptor combination bound thereto; and
(4) has mobility in solution;
(b) permitting hybridization to occur between the target polynucleotide and the capture polynucleotide, wherein mobility of the polymer in solution amplifies hybridization; and
(c) binding the polymer to a solid surface having a receptor member of the ligand/receptor combination bound thereto, wherein the receptor member is capable of binding to the ligand member.

6. The method of Claim 5 wherein the hybridization with polymer amplification is increased about 10-25 fold, as compared to hybridization without polymer.

7. The method of Claim 5 wherein the microbead is a cross-linked dextran microbead, an agarose microbead, a polystyrene microbead, a polytetrafluoroethylene microbead, a polystyrene/latex microbead, a silica microbead, a magnetic microbead, or a microsphere or microbead possessing an activated carboxylate, sulfonate, phosphate or similar activatable group.

8. A target polynucleotide-capturing composition comprising:
(1) a water soluble polymer or a microbead coated with a water soluble polymer, wherein the polymer has at least two copies of a capture polynucleotide covalently bound thereto and wherein the polymer or microbead has mobility in solution, said polymer comprising one member of a ligand receptor pair, and
(2) a solid support being covalently attached to the other member of the ligand receptor pair, so that the interaction of the ligand with its receptor causes the soluble polymer to bind to the solid support.

9. The target polynucleotide-capturing composition of Claim 8 wherein the polymer is selected from the group consisting of polyoxides, polyethers, poly(ethylene imine), acrylic group polymers, vinyl group polymers, polyallyl polymers, polysaccharides, polynucleotides, polypeptides and polyvinylpyrrolidine polymers.

10. The target polynucleotide-capturing composition of Claim 8 wherein the capture polynucleotide is about 15 to about 70 nucleotides in length.

## Patentansprüche

1. Verfahren zum erhöhten Einfangen eines Ziel-Polynucleotides umfassend:
(a) das in Kontakt bringen des Ziel- Polynukleotides mit einem komplementären Einfang- Polynukleotid, das kovalent an ein Polymer gebunden ist,
wobei das Polymer
(1) ein wasserlösliches Polymer ist oder Mikroperlen, die mit einem wasserlöslichen Polymer beschichtet sind;
(2) wenigstens 2 Kopien des Einfang- Polynukleotides hat, die kovalent daran gebunden sind; und
(3) in Lösung Mobilität aufweist;
(b) das Ermöglichen des Auftretens einer Hybridisierung zwischen dem Ziel-Polynukleotid und dem Einfang-Polynukleotid, wobei die Mobilität des Polymers in der Lösung die Hybridisierung verstärkt; und
(c) das Binden des Polymers an eine feste Oberfläche.

2. Verfahren nach Anspruch 1, bei dem die Hybridisierung mit Polymerverstärkung etwa 10 bis 25-fach erhöht wird während einer 10-minütigen Hybridisierung, im Vergleich zu einer Hybridisierung ohne Polymer.

3. Verfahren nach Anspruch 1, bei dem das Polymer ausgewählt wird aus der Gruppe umfassend die Polyoxide, Polyether, Poly (ethyleneimine), Acrylgruppenpolymere, Vinylgruppenpolymere, Polyallylpolymere, Polysaccharide, Polynukleotide, Polypeptide und Polyvinylpyrrolidinpolymere.

4. Verfahren nach Anspruch 1, bei dem das wasserlösliche Polymer ein Molekulargewicht von zwischen etwa 600 und etwa 100 000 dalton aufweist.

5. Verfahren zum erhöhten Einfangen von Ziel-Polynukleotiden umfassend:
(a) das in Kontakt bringen des Ziel- Polynukleotides mit einem komplementären Einfang- Polynukleotid, das kovalent an ein Polymor gebunden ist,
wobei das Polymer
(1) ein wasserlösliches Polymer ist oder Mikroperlen beschichtet mit einem wasserlöslichen Polymer;
(2) wenigstens zwei Kopien des Einfang- Polynukleotides aufweist, die kovalent daran gebunden sind;
(3) einen Liganden einer Ligand/Rezeptorkombination hat, die daran gebunden ist; und
(4) Mobilität in Lösung aufweist;
(b) das Ermöglichen einer auftretenden Hybridisierung zwischen dem Ziel- Polynukleotid und dem Einfang-Polynukleotid, wobei die Mobilität des Polymers in der Lösung die Hybridisierung verstärkt; und
(c) das Binden des Polymers an eine feste Oberfläche die ein Rezeptorglied aus der Ligand/Rezeptorkombination aufweist, die daran gebunden ist, wobei das Rezeptorglied in der Lage ist an den Liganden zu binden.

6. Verfahren nach Anspruch 5, bei dem die Hybridisierung durch Polymerverstärkung etwa 10 bis 25-fach erhöht wird, im Vergleich zu der Hybridisierung ohne Polymer.

7. Verfahren nach Anspruch 5, bei dem die Mikroperlen cross-vernetzte Dextran-Mikroperlen sind, Agarose-Mikroperlen, Polystyrol-Mikroperlen, Polytetrafluorethylen-Mikroperlen, Polystyrol/Latex-Mikroperlen, Siliziumoxid-Mikroperlen, magnetische Mikroperlen, oder Mikrokügelchen oder Mikroperlen die eine aktivierte Carboxylat-, Sulfonat-, Phosphat- oder eine ähnliche aktivierbare Gruppe aufweisen.

8. Ziel-Polynukleotide-einfangende Zusammensetzung umfassend:
(1) ein wasserlösliches Polymer oder eine Mikroperle die mit einem wasserlöslichen Polymer beschichtet ist, wobei das Polymer wenigstens zwei Kopien eines Einfang-Polynukleotides aufweist, kovalent daran gebunden und wobei das Polymer oder die Mikroperle in Lösung Mobilität aufweist, wobei dieses Polymer wenigstens ein Glied eines Ligand/Rezeptorpaars umfaßt, und
(2) einen festen Träger, der kovalent an das andere Glied des Ligand/Rezeptorpaars gebunden ist, so daß die Wechselwirkung des Liganden mit seinem Rezeptor das lösliche Polymer veranlaßt, sich an den festen Träger zu binden.

9. Ziel-Polynukleotide einfangende Zusammensetzung nach Anspruch 8, bei der das Polymer ausgewählt wird aus der Gruppe bestehend aus Polyoxiden, Polyethern, Poly-(ethyleniminen), Acrylgruppenpclymeren, Vinylgruppenpolymeren, Polyallylpolymeren, Polysacchariden, Polynukleotiden, Polypeptiden und Polyvinylpyrrolidinpolymeren.

10. Ziel-Polynukleotide einfangende Zusammensetzung nach Anspruch 8, bei der das Einfang-Polynukleotid etwa 15 bis 70 Nukleotide in der Länge umfaßt.

## Revendications

1. Procédé pour une capture améliorée d'un polynucléotide cible comprenant les étapes consistant à:
(a) mettre en contact le polynucléotide cible avec un polynucléotide de capture complémentaire lié de manière covalente à un polymère,
dans lequel le polymère
(1) est un polymère hydrosoluble ou une microperle enrobée d'un polymère hydrosoluble ;
(2) possède au moins deux copies du polynucléotide de capture liées à lui de manière covalente ; et
(3) possède une mobilité en solution ;
(b) permettre à une hybridation de se produire entre le polynucléotide cible et le polynucléotide de capture, la mobilité du polymère en solution amplifiant l'hybridation ; et
(c) lier le polymère à une surface solide.

2. Procédé selon la Revendication 1 dans lequel l'hybridation avec amplification par le polymère est accrue de 10 à 25 fois pendant une hybridation de 10 minutes, en comparaison d'une hybridation sans polymère.

3. Procédé selon la Revendication 1 dans lequel le polymère est choisi dans le groupe constitué par les polyoxydes, les polyéthers, la poly(éthylèneimine), les polymères du groupe acrylique, les polymères du groupe vinylique, les polymères polyallyliques, les polysaccharides, les polynucléotides, les polypeptides et les polymères de la polyvinylpyrrolidine.

4. Procédé selon la Revendication 1, dans lequel le polymère hydrosoluble possède un poids moléculaire compris entre environ 600 et environ 100 000 daltons.

5. Procédé pour une capture améliorée d'un polynucléotide cible comprenant les étapes consistant à:
(a) mettre en contact le polynucléotide cible avec un polynucléotide de capture complémentaire lié de manière covalente à un polymère,
dans lequel le polymère
(1) est un polymère hydrosoluble ou une microperle enrobée d'un polymère hydrosoluble;
(2) possède au moins deux copies du polynucléotide de capture liées à lui de manière covalente ;
(3) possède un membre ligand d'une combinaison ligand/récepteur lié à lui ; et
(4) possède une mobilité en solution ;
(b) permettre à une hybridation de se produire entre le polynucléotide cible et le polynucléotide de capture, la mobilité du polymère en solution amplifiant l'hybridation ; et
(c) lier le polymère à une surface solide possédant un membre récepteur de la combinaison ligand/récepteur lié à elle, le membre récepteur étant capable de se lier au membre ligand.

6. Procédé selon la Revendication 5, dans lequel l'hybridation avec amplification par le polymère est accrue de 10 à 25 fois, en comparaison d'une hybridation sans polymère.

7. Procédé selon la Revendication 5, dans lequel la microperle est une microperle de dextrane réticulé, une microperle d'agarose, un microperle de polystyrène, une microperle de polytétrafluoroéthylène, une microperle polystyrène/latex, une microperle de silice, une microperle magnétique, ou une microsphère ou une microperle possédant un groupement carboxylate, sulfonate ou phosphate activé ou un groupement activable similaire.

8. Composition pour la capture d'un polynucléotide cible, comprenant :
(1) un polymère hydrosoluble ou une microperle enrobée d'un polymère hydrosoluble, dans lequel le polymère possède au moins deux copies d'un polynucléotide de capture liées à lui de manière covalente et dans lequel le polymère ou la microperle possède une mobilité en solution, ledit polymère comprenant un membre d'une paire ligand-récepteur, et
(2) un support solide fixé de manière covalente à l'autre membre de la paire ligand-récepteur, afin que l'interaction du ligand avec son récepteur provoque la liaison du polymère soluble au support solide.

9. La composition pour la capture d'un polynucléotide cible de la Revendication 8, dans laquelle le polymère est choisi dans le groupe constitué par les polyoxydes, les polyéthers, la poly(éthylèneimine), les polymères du groupe acrylique, les polymères du groupe vinylique, les polymères polyallyliques, les polysaccharides, les polynucléotides, les polypeptides et les polymères de la polyvinylpyrrolidine.

10. La composition pour la capture d'un polynucléotide cible de la Revendication 8, dans laquelle le polynucléotide de capture est d'une longueur d'environ 15 à environ 70 nucléotides.
